# EUROPEAN PATENT SPECIFICATION

(11) **EP 2 420 223 B1**
(45) Date of publication and mention of the grant of the patent: **19.07.2017**
(21) Application number: 11184501.2
(22) Date of filing: 03.03.2009
(51) Int. Cl.: A61K 9/00, A61K 47/02, A61K 47/10, A61K 31/535, A61K 31/5575, A61K 31/045, A61K 31/14, A61K 31/215, A61K 31/785

(54) **Aqueous pharmaceutical compositions containing borate-polyol complexes**
Wässrige pharmazeutische Zusammensetzungen mit Borat-Polyol-Komplexen
Compositions pharmaceutiques aqueuses contenant des complexes de borate-polyol

(30) Priority: 17.03.2008 US 37137 P
(43) Date of publication of application: 22.02.2012
(62) Divisional of application: 09722142.8
(73) Proprietor: NOVARTIS AG, 4056 Basel (CH)
(72) Inventor: Kabra, Bhagwati P., Euless, TX 76039 (US); Jani, Rajni, Fort Worth, TX 76109 (US)
(74) Representative: Kingsbury, Oliver William

(56) References cited:
- EP-A2- 0 462 460
- WO-A1-93/21903
- WO-A1-98/52579
- US-A1- 2005 239 900
- US-B1- 6 743 439
- HOULSBY R D ET AL: "Antimicrobial activity of borate-buffered solutions", ANTIMICROBIAL AGENTS AND CHEMOTHERAPY, AMERICAN SOCIETY FOR MICROBIOLOGY, WASHINGTON, DC, US, vol. 29, no. 5, 1 May 1986 (1986-05-01), pages 803-806, XP003011468, ISSN: 0066-4804

## Description

### Cross-Reference to Related Application

The present application claims priority based on U.S. Provisional Patent Application Serial No. 61/037,137 filed March 17, 2008.

### Technical Field of the Invention

The present invention is related to pharmaceutical compositions that contain borate-polyol complexes for improved preservation of the compositions. More specifically the present invention relates to aqueous multi-dose ophthalmic pharmaceutical compositions containing two or more different polyols in conjunction with borate and a preservative.

### Background of the Invention

The present invention is directed to pharmaceutical compositions formulated so as to have sufficient antimicrobial activity to satisfy the preservation efficacy requirements of the United States Pharmacopeia ("USP") and analogous guidelines in other countries. The ability to achieve preservation is based on a unique combination of formulation components and particularly the use of two or more different polyols in combination with borate.

Many pharmaceutical compositions are required to be sterile (i.e., substantially free of bacteria, fungi and other pathogenic microorganisms). Examples of such compositions include: solutions and suspensions that are injected into the bodies of humans or other mammals; creams, lotions, solutions or other preparations that are topically applied to wounds, abrasions, burns, rashes, surgical incisions, or other conditions where the skin is not intact; and various types of compositions that are applied either directly to the eye (e.g., artificial tears, irrigating solutions, and drug products), or are applied to devices that will come into contact with the eye (e.g., contact lenses).

The foregoing types of compositions can be manufactured under sterile conditions via procedures that are well known to those skilled in the art. However, once the packaging for a product is opened, such that the composition contained therein is exposed to the atmosphere and other sources of potential microbial contamination (e.g., the hands of a human patient), the sterility of the product may be compromised. Such products are typically utilized multiple times by the patient, and are therefore frequently referred to as being of a "multi-dose" nature.

Due to the frequent, repeated exposure of multi-dose products to the risk of microbial contamination, it is necessary to employ a means for preventing such contamination from occurring. The means employed may be: (i) a chemical agent that prevents the proliferation of microbes in a composition, which is referred to herein as an "antimicrobial preservative"; or (ii) a packaging system that prevents or reduces the risk of microbes reaching a pharmaceutical composition within a container.

Prior multi-dose ophthalmic compositions have generally contained one or more antimicrobial preservatives in order to prevent the proliferation of bacteria, fungi and other microbes. Such compositions may come into contact with the cornea either directly or indirectly. The cornea is particularly sensitive to exogenous chemical agents. Consequently, in order to minimize the potential for harmful effects on the cornea, it is preferable to use anti-microbial preservatives that are relatively non-toxic to the cornea, and to use such preservatives at relatively low concentrations.

Balancing the anti-microbial efficacy and potential toxicological effects of anti-microbial preservatives is sometimes difficult to achieve. More specifically, the concentration of an antimicrobial agent necessary for the preservation of ophthalmic formulations from microbial contamination may create the potential for toxicological effects on the cornea and/or other ophthalmic tissues. Using lower concentrations of the anti-microbial agents generally helps to reduce the potential for such toxicological effects, but the lower concentrations may be insufficient to achieve the required level of biocidal efficacy (i.e., antimicrobial preservation).

The use of an inadequate level of antimicrobial preservation may create the potential for microbial contamination. Such contamination is typically undesirable for most biological systems and particularly undesirable for the human eye.

Thus, there is a need for a means of enhancing the activity of anti-microbial agents so that low concentrations of the agents can be utilized without increasing the potential for toxicological effects or subjecting patients to undesirable risks of microbial contamination and resulting ophthalmic infections.

Ophthalmic compositions are generally formulated as isotonic, buffered solutions. Particularly desirable ophthalmic compositions are those containing borate or borate-polyol complexes. Examples of such compositions are disclosed in U.S. Patent Nos. 6,503,497; 6,011,062; 6,849,253; 5,603,929; 5,653,972; 5,849,792; and 5,631,287.

It is generally known that borate-polyol complexes can be used in ophthalmic compositions to enhance anti-microbial activity in the presence of a preservative such as a polymeric quaternary ammonium; see U.S. Patent Nos. 5,505,953; 5,811,466; 6,143,799; and 6,365,636. It has also been shown that increase in amounts of polyol such as sorbitol or mannitol can significantly increase anti-microbial activity even when relatively low amounts of borate are employed. However, mannitol and sorbitol can also affect the resistance to normalization of tear pH after instillation of the compositions in the eye.

Generally, the borate component (e.g., boric acid) of these complexes can provide the ophthalmic composition with significant resistance to normalization of tear pH. It is generally desirable for these ophthalmic compositions to exhibit at least some degree of buffering so that the natural pH of the compositions does not change significantly over time. However, it is also possible for the compositions to exhibit an undesirably high degree of buffering such that, when applied, they can cause tearing of the eye and discomfort to the eye as the eye attempts to maintain its own pH. Thus, it is desirable to minimize the resistance of the compositions to normalization of tear pH after application. The aforementioned polyols, particularly mannitol, sorbitol or both, can significantly enhance the resistance to normalization of tear pH of the borate component. Thus, for the purpose of maintaining desired levels of buffering, it is typically desirable to maintain relatively low concentrations of these polyols in the presence of borate. However, such lower concentrations can limit or lower the anti-microbial activity of the ophthalmic compositions.

U.S. Patent No. 6,743,439 discloses aqueous pharmaceutical solution compositions preserved with a cationic preservative and comprising a cationic drug and a sulfonated styrene/maleic anhydride copolymer. The compositions are said to be particularly well suited for topical ophthalmic use. U.S. Patent Application Publication No. 2005/0239900 discloses the use of low molecular weight amino alcohols in ophthalmic compositions. The travoprost formulation TRAVATAN-Z® was approved by the FDA in September 2006. TRAVATAN-Z® does not contain benzalkonium chloride.

In view of the above, it would be particularly desirable to provide an ophthalmic composition, which includes borate-polyol complex and which exhibits improved buffering, anti-microbial activity, preservative efficacy or any combination thereof.

### Summary of the Invention

The present invention is directed to a multi-dose ophthalmic composition as defined in claim 1 below. The composition is aqueous and preferably satisfies Ph. Eur. A or Ph. Eur. B.

### Detailed Description of the Invention

The present invention is predicated upon the provision of two or more different polyols in the presence of borate for providing a pharmaceutical composition and particularly an ophthalmic composition that exhibits desired buffering and desired anti-microbial activity. Thus, the ophthalmic composition includes a first polyol, a second polyol different from the first polyol and borate. The ophthalmic composition also includes a preservative and can include multiple other ingredients as well. The ophthalmic composition is a multi-dose ophthalmic composition containing travoprost as a therapeutic agent and is configured for topical application as drops directly to the eye.

Unless otherwise indicated, percentages provided for the ingredients of the ophthalmic composition of the present invention are weight/volume (w/v) percentages.

As used herein, the term "borate" shall refer to boric acid, salts of boric acid, borate derivatives and other pharmaceutically acceptable borates, or combinations thereof. Most suitable are: boric acid, sodium borate, potassium borate, calcium borate, magnesium borate, manganese borate, and other such borate salts. Borate interacts with polyols, such as glycerol, propylene glycol, sorbitol and mannitol, to form borate polyol complexes. The type and ratio of such complexes depends on the number of OH groups of a polyol on adjacent carbon atoms that are not in trans configuration relative to each other. It shall be understood that weight/volume percentages of the ingredients polyol and borate include those amounts whether as part of a complex or not.

As used herein, the term "polyol" includes any compound having at least one hydroxyl group on each of two adjacent carbon atoms that are not in *trans* configuration relative to each other. The polyols can be linear or cyclic, substituted or unsubstituted, or mixtures thereof, so long as the resultant complex is water soluble and pharmaceutically acceptable. Examples of such compounds include: sugars, sugar alcohols, sugar acids and uronic acids. Preferred polyols are sugars, sugar alcohols and sugar acids, including, but not limited to: mannitol, glycerin, xylitol, sorbitol and propylene glycol.

As utilized herein, the phrase "less than" relative to a specified concentration (e.g., 1 w/v %) means that the specified component (e.g., antimicrobial preservative) is either not present in the composition at all or is present at a concentration less than the specified limit (e.g., 1 w/v %)). As utilized herein, the phrase "an effective amount of" means that a specified component is present in the composition in an amount sufficient to have an impact on the therapeutic capability, the buffering capability, the preservative capability and/or the anti-microbial capability of the composition.

The compositions of the present invention include a preservative. Potential preservatives include, without limitation, hydrogen peroxide, chlorine containing preservatives such as, but not including, benzalkonium chloride. According to a preferred aspect, however, the ophthalmic composition of the present invention is substantially free of any chloride containing preservatives. The multi-dose ophthalmic composition of the present invention is free of benzalkonium chloride. A preservative included in the ophthalmic composition is a polymeric quaternary ammonium compound.

As used herein, the phrase "substantially free of" as it refers to an ingredient of the ophthalmic composition means that it is contemplated that the ophthalmic solution can be either entirely devoid of that particular ingredient or includes only a nominal amount of that particular ingredient.

The polymeric quaternary ammonium compounds useful in the compositions of the present invention are those which have an antimicrobial effect and which are ophthalmically acceptable. Preferred compounds of this type are described in U.S. Pat. Nos. 3,931,319; 4,027,020; 4,407,791; 4,525,346; 4,836,986; 5,037,647 and 5,300,287; and PCT application WO 91/09523 (Dziabo et al.). The most preferred polymeric ammonium compound is polyquaternium 1, otherwise known as POLYQUAD.RTM. or ONAMERM.RTM. with a number average molecular weight between 2,000 to 30,000. Preferably, the number average molecular weight is between 3,000 to 14,000.

The polymeric quaternary ammonium compounds are used in the compositions of the present invention in an amount that is at least 0.0003 w/v % and more typically greater than about 0.0007 w/v % of the ophthalmic composition. Moreover, the polymeric quaternary ammonium compounds are used in the compositions of the present invention in an amount that is less than about 0.003 w/v % and more typically less than about 0.0015 w/v % of the ophthalmic composition.

As suggested previously, the ophthalmic composition will include a combination of two or more polyols with first polyol being different from second polyol. The first polyol is preferably one that significantly enhances the resistance of the borate component to normalization of tear pH upon instillation of the ophthalmic composition in the eye. In contrast, the second polyol is preferably one that does not or only minimally enhances such resistance of the borate component of the ophthalmic composition.

The first polyol is mannitol ((2R,3R,4R,5R)-hexane-1,2,3,4,5,6-hexol). In a preferred embodiment, the first polyol is entirely or substantially entirely (i.e., at least 95% by weight) mannitol. Of these, it is typically preferred that the first polyol be substantially entirely mannitol.

As used herein, the term "substantially entirely", when used to describe what ingredient[s] are part of a component of the ophthalmic composition, means that it is contemplated that the component is formed entirely of one or more particular ingredient[s] or is formed substantially entirely of those one or more particular ingredient[s] with only a nominal amount of the component being formed of other than those one or more particular ingredients.

The first polyol is at least about 0.25 w/v % of the ophthalmic composition. The first polyol is also less than about 1.5 w/v % and more typically less than about 0.5 w/v % of the ophthalmic composition.

The second polyol can be a single polyol or group of polyols. Polyols for the second polyol include, glycerol (propane-1,2,3-triol), propylene glycol (propane-1,2-diol), or combinations thereof. In a preferred embodiment, the second polyol is entirely or substantially entirely (i.e., at least 95% by weight) glycerol or propylene glycol or both. Of these, it is typically preferred that the second polyol be substantially entirely propylene glycol.

The second polyol is at least about 0.1 w/v %, more typically at least about 0.2 w/v % and even more typically at least about 0.3 w/v % of the ophthalmic composition. The first polyol is also less than about 5 w/v %, more typically less than about 1.8 w/v % and even more typically less than about 1.2 w/v % of the ophthalmic composition.

Generally, it is contemplated that various amounts of borate can be included in the ophthalmic compositions of the present invention. However, it has been found that lower concentrations of borate, when used in combination with the two or more different polyols, can produce unexpectedly superior antimicrobial activity, preservation efficacy, desired buffering or a combination thereof. For the present invention, the borate is at least about 0.25 w/v % of the ophthalmic composition. Furthermore, the borate is less than about 0.5 w/v %, more typically less than about 0.4 w/v %, and even possibly less than about 0.35 w/v % of the ophthalmic composition. This is particularly the case where the combination of polyols and the borate are employed in the presence of a polymeric quaternary ammonium compound (e.g., polyquaternium-1) as preservative.

The resistance to normalization of tear pH of the ophthalmic composition within the eye is typically within a desired range. Such resistance can be quantified in terms of the amount or volume of base or acid per amount or volume of ophthalmic composition used to change the composition pH to a predetermined pH. The amount of base or acid required per amount volume of ophthalmic composition to change the natural pH of the composition to the tear pH (7.5) can be significant since it can represent the resistance the composition will provide to normalize to tear pH after the instillation of the composition in the eye. In particular, for the present invention, resistance to normalization to the tear pH can be quantified as the volume of 1 N NaOH (1 normal NaOH) or 1 N HCl (1 normal HCl) required per volume of ophthalmic composition to change the natural pH of the composition to pH of 7.5. For example, the addition of 10 microliters (µl) of 1 N NaOH may move the pH of one milliliter (ml) of the ophthalmic composition from its natural pH (e.g., pH less than 7.0) to a pH of 7.5. The ophthalmic composition of the present invention may not need any NaOH or HCl to achieve pH of 7.5. Typical ophthalmic compositions of the present invention typically need at least 0.5 µl, more typically at least 1.0 µl, and still more typically at least 2.0 µl of 1 N NaOH to bring one (1) ml of the ophthalmic composition to a pH of 7.5. It is also typical that less than 20 µl, more typically less than 15 µl, still more typically less than 10 µl and even possibly less than 6.0 µl of 1 N NaOH can bring one (1) ml of the ophthalmic composition to a pH of 7.5. Several examples are provided below where resistance to normalization to tear pH has been given as microliters of 1 N NaOH to bring one (1) ml the ophthalmic composition to a pH of 7.5.

The present invention is particularly directed to the provision of multi-dose ophthalmic compositions that have sufficient antimicrobial activity to allow the compositions to satisfy the USP preservative efficacy requirements, as well as other preservative efficacy standards for aqueous pharmaceutical compositions.

The preservative efficacy standards for multi-dose ophthalmic solutions in the U.S. and other countries/regions are set forth in the following table:

### Preservative Efficacy Test ("PET") Criteria (Log Order Reduction of Microbial Inoculum Over Time

| | Bacteria | Fungi |
|---|---|---|
| USP 27 | A reduction of 1 log (90%), by day 7; 3 logs (99.9%) by day 14; and no increase after day 14 | The compositions must demonstrate over the entire test period, which means no increases of 0.5 logs or greater, relative to the initial inoculum. |
| Japan | 3 logs by 14 days; and no increase from day 14 through day 28. | No increase from initial count at 14 and 28 days |
| Ph. Eur. A¹ | A reduction of 2 logs (99%) by 6 hours; 3 logs by 24 hours; and no recovery after 28 days | A reduction of 2 logs (99%) by 7 days, and no increase thereafter |
| Ph. Eur. B | A reduction of 1 log at 24 hours; 3 logs by day 7; and no increase thereafter | A reduction of 1 log (90%) by day 14, and no increase thereafter |
| FDA/ISO 14730 | A reduction of 3 logs from initial challenge at day 14; and a reduction of 3 logs from rechallenge | No increase higher than the initial value at day 14, and no increase higher than the day 14 rechallenge count through day 28. |

| | | |
|---|---|---|
| ¹There are two preservative efficacy standards in the European Pharmacopoeia ' "A" and "B". | | |

The standards identified above for the USP 27 are substantially identical to the requirements set forth in prior editions of the USP, particularly USP 24, USP 25 and USP 26.

The borate/polyol systems described herein may be included in various types of pharmaceutical compositions to enhance anti-microbial activity and preservation of the compositions, such as ophthalmic, otic, nasal and dermatological compositions, but is particularly useful in ophthalmic compositions. Examples of such compositions include ophthalmic pharmaceutical compositions, such as topical compositions used in the treatment of glaucoma, infections, allergies or inflammation. The compositions may be aqueous or non-aqueous, but will generally be aqueous.

The compositions of the present invention may contain various types of therapeutic agents. The invention can include therapeutic agents that are nonionic. Cationic therapeutic agents may also be utilized in the compositions, particularly if the agent is included in the compositions in free base form or in the form of a salt with a monovalent anion, such as a hydrochloride salt.

Examples of therapeutic agents that may be contained in the ophthalmic compositions of the present invention include prostaglandin analogs (e.g., latanoprost, travoprost and unoprostone), hypotensive lipids (e.g., bimatoprost), and glucocorticoids (e.g., prednisolone, dexamethasone and lotoporednol). Examples, which can be in addition to or alternative to the aforementioned, include, without limitation, timolol (e.g., timolol maleate), olopatadine (e.g., olopatadine hydrochloride), brinzolamide, dorzolomide, brimonidine, emadastine, tandospirone, roscovitine, nepafenac, bradykinin, PDE4 inhibitor, combinations thereof or the like. The ophthalmic compositions of the present invention include travoprost.

The present invention can be directed to the provision of multi-dose ophthalmic compositions in connection with the treatment of conditions wherein the cornea or adjacent ocular tissues are irritated, or conditions requiring frequent application of a composition, such as in the treatment of dry eye patients. The compositions may also be particularly useful for treating glaucoma.

The compositions of the present invention will generally be formulated as sterile aqueous solutions. The compositions of the present invention are also formulated so as to be compatible with the eye and/or other tissues to be treated with the compositions. The ophthalmic compositions intended for direct application to the eye will be formulated so as to have a pH and tonicity that are compatible with the eye. It is also contemplated that the compositions can be suspensions or other types of solutions.

The compositions will have a pH in the range of 6.4 to 7.2. The compositions may have an osmolality of 200 to 400 or 450 milliosmoles per kilogram (mOsm/kg), more preferably 240 to 360 mOsm/kg.

The compositions of the present invention may contain various types of pharmaceutical excipients, such as surfactants, viscosity-modifying agents (e.g., hydroxyethyl cellulose (HEC), hydroxypropylmethyl cellulose (HPMC) or a combination thereof) and so on. A surfactant is required. The surfactant for the present invention is Polyoxyethylene (POE) (40) Hydrogenated Castor oil (or PEG (40 Hydrogenated castor oil) (HCO-40).

When an amount, concentration, or other value or parameter is given as either a range, preferred range, or a list of upper preferable values and lower preferable values, this is to be understood as specifically disclosing all ranges formed from any pair of any upper range limit or preferred value and any lower range limit or preferred value, regardless of whether ranges are separately disclosed. Where a range of numerical values is recited herein, unless otherwise stated, the range is intended to include the endpoints thereof, and all integers and fractions within the range. It is not intended that the scope of the invention be limited to the specific values recited when defining a range.

Other embodiments of the present invention will be apparent to those skilled in the art from consideration of the present specification and practice of the present invention disclosed herein.

Table A below provides a listing of exemplary ingredients suitable for an exemplary preferred formulation of the ophthalmic composition of the present invention and a desired weight/volume percentage for those ingredients.

**TABLE A**

| Ingredient | w/v percent |
|---|---|
| travoprost | 0.004 |
| POE 40 Hydrogenated Castor Oil (HCO-40) | 0.5 or 0.1 |
| Boric Acid | 0.3 |
| Propylene Glycol | 0.75 |
| Mannitol | 0.3 |
| Sodium Chloride | 0.35 |
| polymeric quaternary ammonium compound | 0.001 |
| NaOH | sufficient to achieve pH = 6.8 |
| purified water | Q.S. 100 |

It is understood that the weight/volume percents in table A can be varied by ±10%, ± 20%, ±30%, of those weight/volume percents or more and that those variances can be specifically used to create ranges for the ingredients of the present invention. For example, an ingredient weight/volume percent of 10% with a variance of ±20% means that the ingredient can have a weight/volume percentage range of 8 to 12 w/v %.

The following examples are presented to further illustrate selected embodiments of the present invention. The formulations shown in the examples were prepared using procedures that are well-known to persons of ordinary skill in the field of ophthalmic pharmaceutical compositions.

Antimicrobial preservative effectiveness as set forth by the examples infra was determined using an organism challenge test according to the methods described in the United States Pharmacopeia 24 (USP) for category 1A products. Samples were inoculated with known levels of one or more of the following: gram-positive vegetative bacteria (Staphylococcus aureus ATCC 6538), gram-negative vegetative bacteria (Pseudomonas aeruginosa ATCC 9027 and Escherichia coli ATCC 8739), yeast (Candida albicans ATCC 10231) and mold (Aspergillus niger ATCC 16404). The samples were then pulled at specified intervals to determine if the antimicrobial preservative system was capable of killing or inhibiting the propagation of organisms purposely introduced into the formulation. The rate or level of antimicrobial activity determines compliance with the USP preservative efficacy standards for the cited categories of preparations.

**Table B**

| Preservative Standards for U.S. Category 1A Products presented as Log Reduction of Organism Population | | | | | |
|---|---|---|---|---|---|
| Time Pulls | 6 Hours | 24 Hours | 7 days | 14 days | 28 days |
| For Bacteria (S. aureus, P. aeruginosa, and E. coli) | | | | | |
| Ph. Eur. A | 2.0 | 3.0 | NA | NA | NR |
| Ph. Eur. B | NA | 1.0 | 3.0 | NI | NI |
| USP | NA | NA | 1.0 | 3.0 | NI |

| For Fungi (C. albicans and A. niger) | | | | | |
|---|---|---|---|---|---|
| Ph. Eur. A | NA | NA | 2.0 | NA | NI |
| Ph. Eur. B | NA | NA | NA | 1.0 | NI |
| USP | NA | NA | NI | NI | NI |

| | | | | | |
|---|---|---|---|---|---|
| NI = No increase at this or any following time pulls NA = Time point not required for applicable standard (e.g., USP, Ph. Eur. B) NR = No organisms recovered | | | | | |

As shown in Table B, the USP 27 Antimicrobial Effectiveness Test requires that compositions containing Category 1A products have sufficient anti-bacterial activity to reduce an initial inoculum of approximately 10⁵ to 10⁶ bacteria by one log (i.e., a 90% reduction in the microorganism population) over a period of seven (7) days and by three logs (i.e., a 99.9% reduction in the microorganism population) over a period of fourteen (14) days, and requires that there cannot be any increase in the microorganism population following the conclusion of the fourteen day period. Relative to fungi, the USP standards require that the compositions maintain stasis (i.e., no growth) relative to the population of the initial inoculum over the entire 28 day test period. A category 1A product is an injection, or other parenteral including emulsions, otic, sterile nasal products and ophthalmic products made with aqueous bases or vehicles.

The margin of error in calculating microorganism populations is generally accepted to be +/-0.5 logs. Accordingly, the term "stasis", as utilized herein relative to the above-discussed USP standards, means that the initial population cannot increase by more than 0.5 log orders, relative to the initial population.

### EXAMPLES

The formulations of Examples A-U are provided as an illustration of desirability of the present invention. The examples illustrate the antimicrobial activity and/or preservative efficacy of the ophthalmic compositions of the present invention containing the combination of two different polyols particularly in combination with the borate, the polymeric quaternary ammonium compound or both. Percentages of ingredients in Examples A-U are weight/volume percents.

### Examples A through D

Table C provides formulations A through D and data related to those formulations.

**TABLE C**

| Examples | | | A | B | C | D |
|---|---|---|---|---|---|---|
| Travoprost | | Ph. Eur | 0.004 | 0.004 | 0.004 | 0.004 |
| HCO40 | | A | 0.5 | 0.5 | 0.5 | 0.5 |
| Sodium Chloride | | Requir- | 0.72 | 0.69 | 0.66 | None |
| Propylene Glycol | | ements | None | None | None | 1.8 |
| Mannitol | | | 0.1 | 0.3 | 0.9 | 0.3 |
| Boric Acid | | | 0.3 | 0.3 | 0.3 | 0.3 |
| Polyquaternium-1 | | | 0.001 | 0.001 | 0.001 | 0.001 |
| Sodium Hydroxide, Hydrochloric Acid | | | Adjust pH to 6.5 | Adjust pH to 6.5 | Adjust pH to 6.5 | Adjust pH to 6.5 |
| Purified Water | | | QS 100% | QS 100% | QS 100% | QS 100% |
| Resistance to Normalization of tear pH µl/ml | | | 3.6 | 7.2 | 13 | 7.6 |
| S. Aureus | 6 Hours | 2.0 | 1.4 | 1.8 | 2.1 | 5.1 |
| | 24 Hours | 3.0 | 1.9 | 2.7 | 3.0 | 5.1 |
| | 7 Days | | 5.1 | 5.1 | 5.1 | 5.1 |
| | 14 Days | | 5.1 | 5.1 | 5.1 | 5.1 |
| | 28 Days | All | 5.1 | 5.1 | 5.1 | 5.1 |
| Pseudomonas A | 6 Hours | 2.0 | 3.4 | 3.3 | 2.8 | 5.1 |
| | 24 Hours | 3.0 | 3.6 | 4.4 | 3.8 | 5.1 |
| | 7 Days | | 5.1 | 5.1 | 5.1 | 5.1 |
| | 14 Days | | 5.1 | 5.1 | 5.1 | 5.1 |
| | 28 Days. | All | 5.1 | 5.1 | 5.1 | 5.1 |
| E. Coli | 6 Hours | 2.0 | 2.5 | 4.5 | 2.4 | 5.1 |
| | 24 Hours | 3.0 | 5.1 | 5.1 | 4.9 | 5.1 |
| | 7 Days | | 5.1 | 5.1 | 5.1 | 5.1 |
| | 14 Days | | 5.1 | 5.1 | 5.1 | 5.1 |
| | 28 Days. | All | 5.1 | 5.1 | 5.1 | 5.1 |
| Candida A. | 7 Days | 2.0 | 1.0 | 1.3 | 1.4 | 4.9 |
| | 14 Days | NI | 1.5 | 1.9 | 1.9 | 4.9 |
| | 28 Days. | NI | 1.9 | 2.3 | 2.5 | 4.9 |
| A. Niger | 7 Days | 2.0 | 3.0 | 3.0 | 3.7 | 3.5 |
| | 14 Days | NI | 3.5 | 3.7 | 3.6 | 3.7 |
| | 28 Days. | NI | 3.7 | 3.9 | 3.8 | 3.9 |

All four examples A through D contain 0.001% Polyquaternium-1 and 0.3% boric acid. Examples A through C contain only one polyol, mannitol, at a concentration from 0.1%, 0.3% or 0.9%. These three formulations meet only Ph. Eur. B criteria. All of them fail to meet Ph. Eur. A criteria for Candia Albican. In addition Examples A and B fail to meet Ph. Eur. A Criteria for Staph Aureus. Example D which contains a combination of two polyols, 0.3% mannitol and 1.8% propylene glycol, meets Ph. Eur. A criteria.

### Examples E-L

Tables D and E provide formulations E through L and data related to those formulations.

**TABLE D**

| Examples | | E | F | G | H |
|---|---|---|---|---|---|
| Travoprost | | 0.004 | 0.004 | 0.004 | 0.004 |
| HCO40 | | 0.1 | 0.1 | 0.1 | 0.1 |
| Sodium Chloride | | 0.35 | 0.35 | 0.35 | 0.35 |
| Propylene Glycol | | 0.75 | 0.75 | 0.75 | 0.75 |
| Mannitol | | 0.3 | 0.3 | 0.3 | None |
| Boric Acid | | 0.3 | 0.3 | None | 0.3 |
| Polyquaternium-1 | | 0.001 | None | 0.001 | 0.001 |
| Sodium Hydroxide, Hydrochloric Acid | | Adjust pH to 6.8 | Adjust pH to 6.8 | Adjust pH to 6.8 | Adjust pH to 6.8 |
| Purified Water | | QS 100% | QS 100% | QS 100% | QS 100% |
| Resistance to Normalization of tear pH µl/ml | | 5.6 | - | 0.9 | 1.6 |
| S. Aureus 6 Hours | 2.0 | 4.0 | 0.0 | 1.8 | 3.6 |
| 24 Hours | 3.0 | 4.9 | 0.0 | 2.0 | 5.0 |
| 7 Days | | 4.9 | 0.5 | 5.0 | 5.0 |
| 14 Days | | 4.9 | 2.1 | 5.0 | 5.0 |
| 28 Days | All | 4.9 | 4.4 | 5.0 | 5.0 |
| Pseudomonas A 6 Hrs | 2.0 | 5.0 | 0.2 | 2.6 | 4.9 |
| 24 Hours | 3.0 | 5.0 | 0.3 | 4.5 | 4.9 |
| 7 Days | | 5.0 | 0.6 | 4.9 | 4.9 |
| 14 Days | | 5.0 | 0.9 | 4.9 | 4.9 |
| 28 Days. | All | 5.0 | 1.2 | 4.9 | 4.9 |
| E. Coli 6 Hours | 2.0 | 5.0 | 0.1 | 3.3 | 5.0 |
| 24 Hours | 3.0 | 5.0 | 0.0 | 5.0 | 5.0 |
| 7 Days | | 5.0 | 0.0 | 5.0 | 5.0 |
| 14 Days | | 5.0 | 0.0 | 5.0 | 5.0 |
| 28 Days. | All | 5.0 | 0.4 | 5.0 | 5.0 |
| Candida A. 7 Days | 2.0 | 4.6 | 0.3 | 2.9 | 4.9 |
| 14 Days | NI | 4.9 | 0.3 | 4.3 | 4.9 |
| 28 Days. | NI | 4.9 | 0.7 | 4.9 | 4.9 |
| A. Niger 7 Days | 2.0 | 3.0 | 3.0 | 0.1 | 1.1 |
| 14 Days | NI | 3.6 | 3.6 | 0.6 | 1.1 |
| 28 Days. | NI | 3.6 | 2.9 | 0.6 | 1.0 |

**TABLE E**

| Examples | | I | J | K | L |
|---|---|---|---|---|---|
| Travoprost | | 0.004 | 0.004 | 0.004 | 0.004 |
| HCO40 | | 0.1 | 0.1 | 0.1 | 0.1 |
| Sodium Chloride | | 0.35 | 0.66 | None | None |
| Propylene Glycol | | None | None | None | 0.75 |
| Mannitol | | 2.3 | 0.3 | 4.6 | 2.3 |
| Boric Acid | | 0.3 | 0.3 | 0.3 | 0.3 |
| Polyquaternium-1 | | 0.001 | 0.001 | 0.001 | 0.001 |
| Sodium Hydroxide, Hydrochloric Acid | | Adjust pH to 6.8 | Adjust pH to 6.8 | Adjust pH to 6.8 | Adjust pH to 6.8 |
| Purified Water | | QS 100% | QS 100% | QS 100% | QS 100% |
| Resistance to Normalization of tear pH µl/ml | | - | 6.2 | 2.5 | 8.7 |
| S. Aureus 6 Hours | 2.0 | 2.7 | 2.0 | 4.9 | 4.9 |
| 24 Hours | 3.0 | 3.9 | 2.9 | 4.9 | 4.9 |
| 7 Days | | 4.9 | 4.9 | 4.9 | 4.9 |
| 14 Days | | 4.9 | 4.9 | 4.9 | 4.9 |
| 28 Days | All | 4.9 | 4.9 | 4.9 | 4.9 |
| Pseudomonas A 6 Hrs | 2.0 | 3.7 | 2.5 | 4.8 | 4.8 |
| 24 Hours | 3.0 | 4.8 | 4.3 | 4.8 | 4.8 |
| 7 Days | | 4.8 | 5.0 | 4.8 | 4.8 |
| 14 Days | | 4.8 | 5.0 | 4.8 | 4.8 |
| 28 Days. | All | 4.8 | 5.0 | 4.8 | 4.8 |
| E. Coli 6 Hours | 2.0 | 4.1 | 3.1 | 4.2 | 4.8 |
| 24 Hours | 3.0 | 4.8 | 4.9 | 4.8 | 4.8 |
| 7 Days | | 4.8 | 4.9 | 4.8 | 4.8 |
| 14 Days | | 4.8 | 4.9 | 4.8 | 4.8 |
| 28 Days. | All | 4.8 | 4.9 | 4.8 | 4.8 |
| Candida A. 7 Days | 2.0 | 3.3 | 1.0 | 4.2 | 5.0 |
| 14 Days | NI | 3.5 | 1.3 | 5.0 | 5.0 |
| 28 Days. | NI | 4.6 | 3.0 | 5.0 | 5.0 |
| A. Niger 7 Days | 2.0 | 1.8 | 3.6 | 0.1 | 2.0 |
| 14 Days | NI | 2.7 | 3.7 | 0.9 | 2.6 |
| 28 Days. | NI | 2.9 | 3.6 | 0.9 | 3.0 |

Example E is a representative example of this invention. It contains lower concentrations of boric acid (0.3%) and mannitol (0.3%). It has a preferred propylene glycol concentration (0.75%). This formulation is also isotonic and meets pH. Eur A preservation.

Example F has the same composition as example E except that it does not contain Polyquaternium-1 and is substantially without any conventional preservative and rather the antimicrobial activity is provided by a system that consists or consists essentially of borate and a combination of polyols. It fails USP, Ph. Eur B and Ph. Eur. A preservation, however, has good activity against A. Niger. Thus, Polyquaternium-1 is typically desirable for the present invention.

Example G has the same composition as example E except that it does not contain boric acid. It meets USP, preservation criteria but fails to meet both Ph. Eur B and Ph. Eur. A preservation criteria. This removal of boric acid significantly affects microbial activity against A. Niger. It also reduces activity against S. Aureus. Thus boric acid is desirable for the ophthalmic composition of the present invention.

Example H has the same composition as example E except that it does not contain mannitol. It meets USP and Ph. Eur B preservation criteria but fails to meet Ph. Eur. A preservation criteria. Removal of mannitol significantly affects microbial activity against A. Niger. It is believed that Mannitol itself does not have any activity against A. Niger as shown by Example G. However, at a lower concentration its complex with boric acid has very significant activity against A. Niger. Thus, it is desirable for the ophthalmic composition of the present invention to have at least a low concentration of mannitol.

Examples I, J, and K do not contain propylene glycol. In example I, propylene glycol was replaced by additional mannitol. Increase in mannitol to boric acid ratio significantly increases complexation and ionization of boric acid. However, it is believed that the activity of boric acid-polyol complex against A. Niger increases with lower level of ionization/complexation of boric acid and that the activity starts decreasing as complexation/ionization of boric acid increases further. As a result, the microbial activity of Example J against A. Niger is higher than Example H, but is lower than Example E. The composition of Example I does not meet Ph. Eur. A PET criteria. Furthermore, this increase in ionization of boric acid increases resistance to normalization of tear pH and hence, is not desirable beyond a point. Thus, for the present invention, it is generally preferred to keep mannitol concentration below about 1.5%. Higher mannitol concentrations are typically not desirable.

In example J, propylene glycol has been replaced by additional amount of sodium chloride. For this example, removal of propylene glycol affects candida albicans and Staph Aureus. However, A. Niger activity is not significantly affected. The formulation meets USP and Ph. Eur. B Criteria but fails Ph. Eur. A criteria.

In example K, both propylene glycol and sodium chloride are replaced with mannitol. Thus formulation has a high concentration of mannitol (4.6%). Such a high concentration of mannitol at 0.3% boric acid provides significantly enhanced activity of Polyquatenium-1 against candida albicans and staph aureus, however has relatively poor activity against A. Niger. Thus, a high concentration of mannitol alone is typically not sufficient to provide Ph. Eur A or even Ph. Eur. B preservation.

In example L, sodium chloride is replaced with additional mannitol. Thus mannitol concentration is 2.3%. Formulation also has 0.75% propylene glycol. It meets Ph. Eur. A preservation. However, its activity against A. Niger is slightly less than that of Examples E with 0.3% boric cid. Thus, it is believed that activity against A. Niger decreases beyond a certain concentration of mannitol as a higher amount of boric acid is complexed. Thus it is typically preferred to keep mannitol concentration below 1.5 w/v %.

With reference to previous example D, there is additional propylene glycol instead of sodium chloride. This formulation passes Ph. Eur A. and has good activity against A. Niger. Thus, unlike mannitol, it is believed that higher concentration of propylene glycol does not typically reduce microbial activity as it does not complex with boric acid to same extent.

### Examples M-P

Table F provides formulations M through P and data related to those formulations.

**TABLE F**

| Examples | | | M | N | O | P |
|---|---|---|---|---|---|---|
| Travoprost | | | 0.002 | 0.002 | 0.004 | 0.002 |
| HCO40 | | | 0.1 | 0.1 | 0.1 | 0.1 |
| Sodium Chloride | | | 0.66 | 0.60 | 0.46 | 0.35 |
| Propylene Glycol | | | None | 0.25 | 0.5 | 0.75 |
| Mannitol | | | 0.3 | 0.3 | 0.3 | 0.3 |
| Boric Acid | | | 0.3 | 0.3 | 0.3 | 0.3 |
| Polyquaternium-1 | | | 0.001 | 0.001 | 0.001 | 0.001 |
| Sodium Hydroxide, Hydrochloric Acid | | | Adjust pH to 6.8 | Adjust pH to 6.8 | Adjust pH to 6.8 | Adjust pH to 6.8 |
| Purified Water | | | QS 100% | QS 100% | QS 100% | QS 100% |
| Resistance to Normalization of tear pH µl/ml | | | 7.4 | - | - | 6.8 |
| S. Aureus | 6 Hours | 2.0 | 2.0 | 1.8 | 3.0 | 3.2 |
| | 24 Hours | 3.0 | 3.0 | 3.0 | 4.2 | 4.9 |
| | 7 Days | | 4.9 | 4.9 | 4.9 | 4.9 |
| | 14 Days | | 4.9 | 4.9 | 4.9 | 4.9 |
| | 28 Days | All | 4.9 | 4.9 | 4.9 | 4.9 |
| Pseudomonas | A 6 Hours | 2.0 | 5.0 | 4.8 | 3.5 | 5.0 |
| | 24 Hours | 3.0 | 5.0 | 5.0 | 4.8 | 5.0 |
| | 7 Days | | 5.0 | 5.0 | 4.8 | 5.0 |
| | 14 Days | | 5.0 | 5.0 | 4.8 | 5.0 |
| | 28 Days. | All | 5.0 | 5.0 | 4.8 | 5.0 |
| E. Coli | 6 Hours | 2.0 | 2.3 | 2.8 | 4.4 | 4.5 |
| | 24 Hours | 3.0 | 4.6 | 4.9 | 4.8 | 4.9 |
| | 7 Days | | 4.9 | 4.9 | 4.8 | 4.9 |
| | 14 Days | | 4.9 | 4.9 | 4.8 | 4.9 |
| | 28 Days. | All | 4.9 | 4.9 | 4.8 | 4.9 |
| Candida A. | 7 Days | 2.0 | 1.3 | 2.4 | 5.0 | 5.0 |
| | 14 Days | NI | 1.5 | 2.3 | 5.0 | 5.0 |
| | 28 Days. | NI | 2.6 | 2.4 | 5.0 | 5.0 |
| A. Niger | 7 Days | 2.0 | 3.1 | 3.7 | 3.0 | 3.7 |
| | 14 Days | NI | 3.7 | 3.7 | 3.1 | 3.7 |
| | 28 Days. | NI | 3.1 | 3.7 | 3.1 | 3.6 |

Examples M to P show the effect of propylene glycol concentration. The results show that 0.25% propylene glycol significantly improves preservation against candida albicans. 0.5% propylene glycol further improves preservation against Staph Aureus and Candida Albicans. Thus, Propylene glycol concentrations 0.3% and higher are typically needed to produce desired results and propylene glycol concentrations 0.5% and higher are typically preferred.

### Example Q

Table G provides formulation Q and data related to that formulation.

**TABLE G**

| | | | |
|---|---|---|---|
| Examples | | | Q |
| Travoprost | | | 0.004 |
| HCO40 | | | 0.1 |
| Sodium Chloride | | | 0.35 |
| Propylene Glycol | | | 0.75 |
| Mannitol | | | 0.3 |
| Boric Acid | | | 0.3 |
| Polyquaternium-1 | | | 0.001 |
| Sodium Hydroxide, Hydrochloric Acid | | | Adjust pH to 7.4 |
| Purified Water | | | QS 100% |
| S. Aureus | 6 Hours | 2.0 | 4.9 |
| | 24 Hours | 3.0 | 4.9 |
| | 7 Days | | 4.9 |
| | 14 Days | | 4.9 |
| | 28 Days | All | 4.9 |
| Pseudomonas A | 6 Hours | 2.0 | 5.0 |
| | 24 Hours | 3.0 | 5.0 |
| | 7 Days | | 5.0 |
| | 14 Days | | 5.0 |
| | 28 Days. | All | 5.0 |
| E. Coli | 6 Hours | 2.0 | 5.0 |
| | 24 Hours | 3.0 | 5.0 |
| | 7 Days | | 5.0 |
| | 14 Days | | 5.0 |
| | 28 Days. | All | 5.0 |
| Candida A. | 7 Days | 2.0 | 4.9 |
| | 14 Days | NI | 4.9 |
| | 28 Days. | NI | 4.9 |
| A. Niger | 7 Days | 2.0 | 2.8 |
| | 14 Days | NI | 3.4 |
| | 28 Days | NI | 2.8 |

As mentioned earlier, Example E of table D is a representative example of this invention. It contains lower concentrations of boric acid (0.3%) and mannitol (0.3%). It has the preferred propylene glycol concentration (0.75%). Example Q has the same composition, except that it has a pH of 7.4 instead of pH 6.8. The formulation Q also meets Ph. Eur A preservation.

### Examples R-U

Table H provides formulations R-U and data related to those formulations.

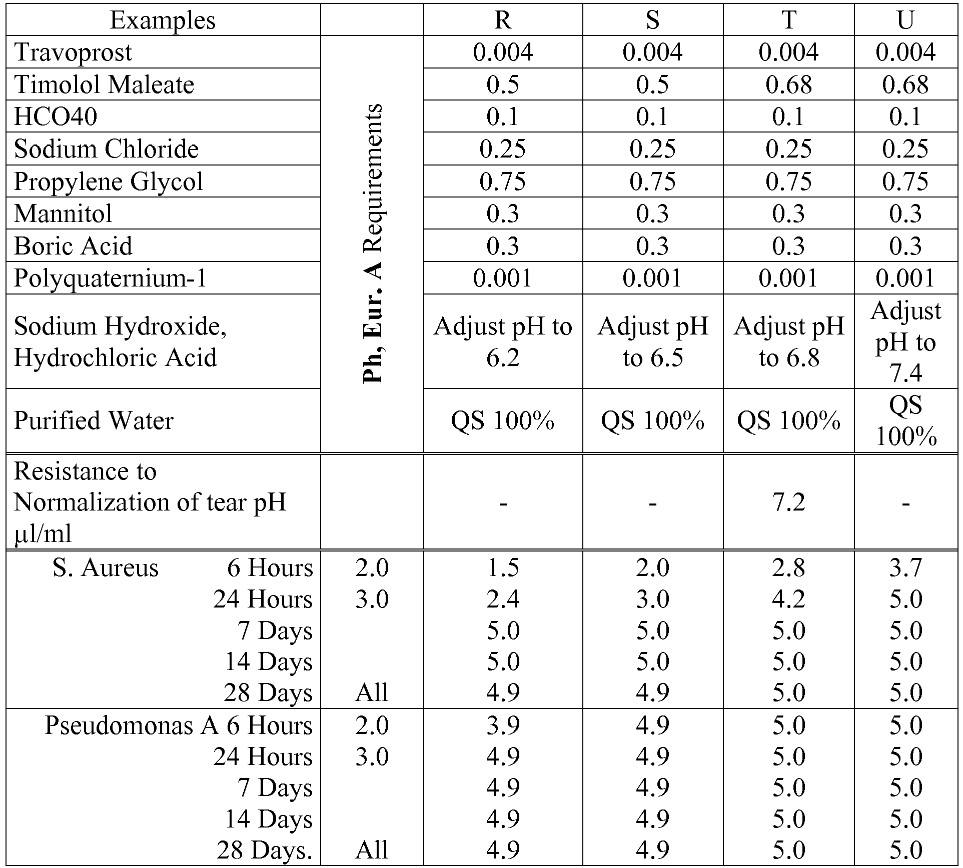

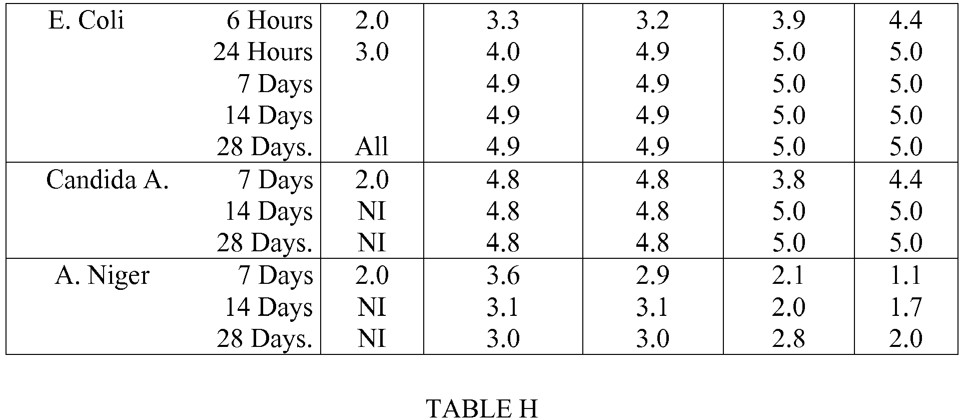

Example T is similar to example E except that it contains timolol maleate and lower concentration of sodium chloride. Addition of timolol maleate, which has multivalent maleate ions, has slightly adverse effect on the preservative performance. However, it still meets Ph. Eur. A Activity. However formulations (R and U) at extreme pH's of 6.2 and 7.4 meet Ph. Eur. B criteria but fail Ph. Eur A criteria for staph aureus and Aspergillus niger, respectively.

## Claims

1. A multi-dose ophthalmic composition configured for topical application as drops directly to the eye, comprising:
first polyol, the first polyol being mannitol, wherein the first polyol is at least 0.25 but less than 1.5 w/v % of the composition;
second polyol, the second polyol being selected from propylene glycol, glycerine or a combination thereof, wherein the second polyol is at least 0.1 but less than 5 w/v % of the composition;
borate, wherein the borate is at least 0.25 w/v % of the composition but less than 0.5 w/v % of the composition;
antimicrobial preservative wherein the preservative is at least 0.0003 but less than 0.003 w/v % of the composition and wherein the preservative is a polymeric quaternary ammonium compound;
travoprost;
polyoxyethylene (40) hydrogenated castor oil wherein the polyoxyethylene (40) hydrogenated castor oil is at least 0.03 but less than 0.5 w/v % of the composition; and
water,
wherein the pH of the composition is from 6.4 to 7.2; and
wherein the composition is substantially free of any benzalkonium chloride.

2. A composition as in claim 1 wherein the composition satisfies Ph. Eur. A, Ph. Eur. B or both.

3. A composition as in claim 1 or 2 wherein the preservative includes polyquaternium-1.

4. A composition as in claim 1 or 2 wherein the composition is substantially free of any chlorine containing agents.

5. A composition as in any of claims 1-4 wherein the resistance provided by the composition to normalization of tear pH after instillation in the eye is less than 15 µl of 1 M NaOH/mL of composition, preferably less than 10 µl of 1 M NaOH/mL of composition.

6. A composition as in any of claims 1-5 wherein the second polyol is propylene glycol.

7. A composition as in any of claims 1-6 further comprising sodium chloride.

## Patentansprüche

1. Ophthalmische Mehrfachdosis-Zusammensetzung konfiguriert zur topischen Anwendung als Tropfen direkt in das Auge, umfassend:
ein erstes Polyol, wobei das erste Polyol Mannitol ist, wobei das erste Polyol mindestens 0,25, aber weniger als 1,5 % (w/v) der Zusammensetzung ausmacht;
ein zweites Polyol, wobei das zweite Polyol ausgewählt ist aus Propylenglycol, Glycerin oder einer Kombination aus diesen, wobei das zweite Polyol mindestens 0,1, aber weniger als 5 % (w/v) der Zusammensetzung ausmacht;
ein Borat, wobei das Borat mindestens 0,25 % (w/v) der Zusammensetzung, aber weniger als 0,5 % (w/v) der Zusammensetzung ausmacht;
ein antimikrobielles Konservierungsmittel, wobei das Konservierungsmittel mindestens 0,0003, aber weniger als 0,003 % (w/v) der Zusammensetzung ausmacht und wobei das Konservierungsmittel eine polymere quartäre Ammoniumverbindung ist;
Travoprost;
hydriertes Polyoxyethylen- (40) Rizinusöl, wobei das hydrierte Polyoxyethylen- (40) Rizinusöl mindestens 0,03, aber weniger als 0,5 % (w/v) der Zusammensetzung ausmacht; und
Wasser,
wobei der pH-Wert der Zusammensetzung zwischen 6,4 und 7,2 liegt; und
wobei die Zusammensetzung im Wesentlichen frei von jedem Benzalkoniumchlorid ist.

2. Zusammensetzung nach Anspruch 1, wobei die Zusammensetzung Ph. Eur. A, Ph. Eur. B oder beiden entspricht.

3. Zusammensetzung nach Anspruch 1 oder 2, wobei das Konservierungsmittel Polyquaternium-1 umfasst.

4. Zusammensetzung nach Anspruch 1 oder 2, wobei die Zusammensetzung im Wesentlichen frei von allen chlorhaltigen Mitteln ist.

5. Zusammensetzung nach einem der Ansprüche 1-4, wobei der von der Zusammensetzung gebotene Widerstand gegen die Normalisierung des pH-Wertes der Tränenflüssigkeit nach Instillation in das Auge weniger als 15 µl von 1 M NaOH/ml der Zusammensetzung beträgt, vorzugsweise weniger als 10 µl von 1 M NaOH/ml der Zusammensetzung.

6. Zusammensetzung nach einem der Ansprüche 1-5, wobei das zweite Polyol Propylenglycol ist.

7. Zusammensetzung nach einem der Ansprüche 1-6, weiterhin umfassend Natriumchlorid.

## Revendications

1. Composition ophtalmique multidose conçue pour une application topique sous forme de gouttes directement dans l'oeil, comprenant :
un premier polyol, le premier polyol étant un mannitol, le premier polyol représentant au moins 0,25, mais moins de 1,5 % (p/v) de la composition ;
un second polyol, le second polyol étant choisi parmi le propylèneglycol, la glycérine ou une combinaison de ceux-ci, le second polyol représentant au moins 0,1, mais moins de 5 % (p/v) de la composition ;
un borate, le borate représentant au moins 0,25 % (p/v) de la composition, mais moins de 0,5 % (p/v) de la composition ;
un agent de conservation antimicrobien, l'agent de conservation représentant au moins 0,0003, mais moins de 0,003 % (p/v) de la composition et l'agent de conservation étant un composé d'ammonium quaternaire polymère ;
du travoprost ;
de l'huile de ricin hydrogénée de polyoxyéthylène (40), l'huile de ricin hydrogénée de polyoxyéthylène (40) représentant au moins 0,03, mais moins de 0,5 % (p/v) de la composition ; et
de l'eau,
dans laquelle le pH de la composition varie de 6,4 à 7,2 ; et
dans laquelle la composition est sensiblement exempte de tout chlorure de benzalkonium.

2. Composition selon la revendication 1, dans laquelle la composition satisfait la Ph. Eur. A, la Ph. Eur. B ou les deux.

3. Composition selon la revendication 1 ou 2, dans laquelle l'agent de conservation comprend du polyquaternium-1.

4. Composition selon la revendication 1 ou 2, dans laquelle la composition est sensiblement exempte de tout agent contenant du chlore.

5. Composition selon l'une quelconque des revendications 1 à 4, dans laquelle la résistance procurée par la composition à la normalisation du pH du larmoiement après instillation dans l'oeil est inférieure à 15 µl de NaOH 1M/ml de composition, de préférence moins de 10 µl de NaOH 1M/ml de composition.

6. Composition selon l'une quelconque des revendications 1 à 5, dans laquelle le second polyol est le propylèneglycol.

7. Composition selon l'une quelconque des revendications 1 à 6 comprenant en outre du chlorure de sodium.
